# EUROPEAN PATENT SPECIFICATION

(11) **EP 3 355 828 B1**
(45) Date of publication and mention of the grant of the patent: **13.11.2019**
(21) Application number: 16775646.9
(22) Date of filing: 28.09.2016
(51) Int. Cl.: A61C 17/02, A61C 1/00, A61B 18/22

(54) **DEVICE FOR STERILIZING TREATMENTS, EQUIPMENT COMPRISING THE DEVICE**
VORRICHTUNG FÜR STERILISIERUNGSBEHANDLUNGEN, AUSRÜSTUNG MIT DER VORRICHTUNG
DISPOSITIF POUR TRAITEMENTS DE STÉRILISATION, ÉQUIPEMENT COMPRENANT LEDIT DISPOSITIF

(30) Priority: 29.09.2015 IT UB20153986
(43) Date of publication of application: 08.08.2018
(73) Proprietor: El.En. S.p.A., 50041 Calenzano (Fl) (IT)
(72) Inventor: MASOTTI, Leonardo, 50019 Sesto Fiorentino (FI) (IT); CALVANI, Paolo, 50133 Firenze (IT); BRESCHI, Luca, 59021 Vaiano (PO) (IT)
(74) Representative: Mannucci, Michele
(86) International application number: PCT/EP2016/073134
(87) International publication number: WO 2017/055362

(56) References cited:
- EP-A1- 0 411 132
- WO-A1-2007/007331
- WO-A1-2009/039456
- DE-A1-102014 203 920
- JP-A- 2003 001 465
- US-A1- 2002 077 623
- US-A1- 2004 068 256
- US-A1- 2010 312 054
- US-B1- 7 320 594

## Description

### Technical field

The present invention relates to medical devices and equipment. Embodiments described herein particularly relates to devices and equipment to be used for treating radicular canals, gingival pockets, or other pathologic conditions of the mouth and other body parts, also affected by sores, wounds and prostheses with percutaneous shunts.

### Base of the invention

Teeth are comprised of an outer shell comprising the outer enamel layer and an inner dentine layer. Inside said shell A cavity, called pulp cavity or neural cavity, is arranged in said shell. The cavity contains a system of blood vessels, lymphatic vessels and nerves, as well as the pulp, extending from the bone through a network of radicular canals, also called root canals. The root canals travel the whole teeth root, at the end of which a foramen connects to the paradontium. The vascular-nervous bundle ensuring nutriment and sensitivity to the tooth is located inside the root canal.

Many dental interventions often relate to the root canal treatment. If the pulp cavity is infected, for example due to a caries, or is damaged following a trauma, the root canal treatment is often the only way to save the tooth. A serious caries or other injury can cause damages or infections to the tooth pulp. In the root canal treatment, also called endodontics, the dentist removes the damaged or infected pulp and replaces it with a special filling material that contributes to keep the remaining structure of the tooth.

Currently, in order to treat the root canals, endodontics intervention is performed, making an opening in the tooth crown to access the pulp cavity. The blood vessels, the nerve, the pulp and, if necessary, part of the dentine are removed through mechanical systems. The cavity thus obtained is then sealed. In some cases, in this cavity a pin is inserted to support a prosthesis replacing the tooth crown.
the cleaning process of the root canals through mechanical means is complex due to the fact that it is difficult to access the teeth, especially the molar teeth, and that the root canals are tortuous and have small dimensions, especially in the deepest area, inside the roots.

An incomplete cleaning of the root canals can have serious and even very serious consequences, including heart infections.

Systems using laser radiation have been studied to facilitate root canal treatment. For example, US5968039 discloses a dental tool using an optical fiber to convey a laser radiation into the root canal. The distal end of the fiber is so processed as to emit a laser beam onto an annular surface surrounding the fiber axis, instead of the apical area thereof, in order to better irradiate the side walls of the root canal. However, this solution is not satisfactory, as the laser radiation does not completely achieve the inside of the root canal.

The currently used techniques for root canal treatment are unsatisfactory as regards the elimination of pathogens (bacteria) that can remain inside the treated area with serious consequences for the patient.

To kill the microorganisms, it is necessary to locally or systemically apply antibiotics. The use of antibiotics have some inconveniences, like, first of all, the development of resistant microorganisms and, secondly, side effects due to patient's intolerance to given antibiotics.

With further reference to the dental field, a further pathological condition requiring antibacterial treatments is the formation of gingival pockets due to an increase in the gingival sulcus. The gingival sulcus is a narrow and shallow canal, positioned at the sides of the tooth and delimited at one side by the tooth surface and, at the other side, by the sulcus epithelium of the marginal gingiva. Under normal conditions, the depth of this sulcus is comprised between 1 and 3 millimeters, but it increases up to 4 millimeters and more in case of periodontopathies. Under some pathological conditions, the depth of the gingival sulcus may increase, thus forming a so-called gingival pocket. The main cause therefor is the formation of plaque that, if not removed, causes the destruction of the epithelium that moves to a deeper area, causing an increase in the depth of the gingival sulcus and the formation of the pocket.

Microorganisms in the gingival pockets produce bacterial toxins causing gingivitis. The inflammation of the gingival tissue causes a displacement of the same tissue, that moves away from its original seat, leaving the roots exposed in a unsightly and often sensible way. After the gum has been moved away from the tooth, there is also a resorption of the bone, with the consequent formation of bony pockets where the plaque accumulates more easily and transforms into tartar due to calcification.

The bacteria that have not been removed from teeth and gums remain in the pockets and produce toxins killing the osteoblasts, with a consequent bone resorption causing movement and even falling out of the teeth, if not suitably treated. The risk of tooth falling out is not actually linked to the gingival pocket, but to the bone resorption that occurs in case treatment is not performed (due to the bacterial infection).

The therapy of gingival pockets and parodontopathies depends on the disease stage. Mucogingival surgery comprises the procedures suitable to correct defects in morphology, position and/or quantity of the parodontal soft tissues (gums). The main applications are the covering of the exposed root canals by stretching the existing gum, up to the gum graft, wherein, in case of large gingival pockets to be covered, the gum is taken from the palate. These interventions are very invasive.

There is therefore the need, in the dental field, for tools and methods suitable to efficiently intervene on the gingival pockets, eliminating the microorganisms inside them, in order to prevent the aggravation of this pathology that can lead to the above described consequences affecting the bone.

In the dental field, microorganism can proliferate also in other areas, for instance the areas surround the pins of dental implants and prostheses, the areas near surgery metal stitches etc.

Similar problems can occur when a foreign element, for instance a metal stitch, or a prosthesis, extend through the skin and enters the tissues below. This kind of problems can occur, for example, when applying metal stitches to the breast bone during heart surgery. In all these cases, the elimination of bacterial is fundamental for the patient's health; currently, the most common approach is based on the use of antibiotics, with the limits and drawbacks mentioned above.

US 2002/077623 A1 discloses a medical equipment for tissue laser treatments, a first duct, a light guide, a laser source and a transmissive tube.

EP 0 411 132 A1 discloses a liquid comprising scattering particles which stays inside a balloon.

There is therefore a need for more efficient device and method for treating the above mentioned situations.

### Summary of the Invention

The present invention is set out in the appended claims.

According to a first aspect, a medical device is provided for laser treatment of tissues, comprising a first duct having a proximal end, associated with a hand-piece, and an open distal end, the first duct communicating with an inlet port for a liquid containing scattering particles. The device furthermore comprises a light guide, arranged and configured to convey a laser radiation, coming from a suitable laser source, near the open distal end of the first duct. The scattering particles may be solid, liquid or gaseous, and are so configured as to scatter the laser radiation, conveyed by the light guide, near the open distal end of the first duct. In particular, the dimension and the material of the particles may be selected, for example, according to the wavelength of the laser radiation, in order to optimize the scattering effect the particles have on the radiation conveyed by the light guide.

In embodiments described herein, the medical device comprises a liquid source, in said liquid scattering particles being suspended, the source being in fluid connection with the first duct.

The light guide may be arranged outside the first duct, but it is preferably housed inside the first duct and extends longitudinally there along up to an area adjacent to the open distal end of the first duct. In this way, the light guide is protected and the overall dimensions of the device are reduced.

If the light guide is comprised of an optical fiber, the device may have a particularly compact structure. The light guide may be constituted by a single optical fiber, or by a plurality of optical fibers forming a bundle. In the last case, the optical fibers may be equal to, or different from, one another as regards nature, dimensions or other features. For example, different optical fibers may be used to convey laser radiations at different wavelengths. It is also possible to convey, to the area to be treated, not only a curative radiation, for example a sterilizing and/or bio-stimulating radiation, but also a radiation just in order to lighting up, thus facilitating the treatment.

The light guide may be arranged in the duct, or adjacent thereto, in an adjustable manner, in order to adjust the distance between the end of the light guide and the end of the duct. In this way, the light guide may project from the duct by an adequate distance, or it may be completely housed inside the duct.

According to an improved embodiment, the device furthermore comprises a second suction duct for removing the liquid from a treated area. The second suction duct may have an inlet near the open distal end of the first duct, so as to remove liquid and scattering particles from the treated area. This allows, for example, reducing liquid dispersion, making the treatment more comfortable and reducing the trouble for the patient. Moreover, sucking of the liquid contributes to remove heat and/or residues from the treated area.

A separate pump or a pump integrated in the hand-piece may be used to pump the liquid with the scattering particles. In some particularly advantageous embodiments, the pump may be configured and arranged to pump liquid with scattering particles into the first duct and to suck liquid with scattering particles through the second suction duct. The pump may be a peristaltic pump, for instance.

The combined use of liquid with scattering particles and of laser radiation conveyed by the light guide allows laser light to radiate areas that cannot be directly accessed by the radiation coming directly from the light guide. In fact, the laser radiation emitted by the light guide, for example by the free end of an optical fiber, propagates according to a rectilinear path. Therefore, it is not able to achieve areas that are in shadow with respect to the light emitting point. This can be a serious limit for example when using laser in some surgical or dental applications. Typically, serious limits can occur in cleaning the root canals, due to the presence of recesses and areas that are difficult to be accessed or that can be accessed only through devious paths, where the radiation emitted by a fiber cannot propagate. The scattering particles conveyed by the liquid to the laser radiation emitting area cause the radiation to scatter according to an infinity of directions, thus achieving, for example, also shadow areas, complex paths and undercut areas. Spraying a free liquid inside the treated volume, for example a root canal, allows the laser radiation completely to radiate substantially the entire surface that is wet by the liquid.

According to a further aspect, the invention relates to a medical equipment comprising a device as defined above, a laser source that can be connected to the light guide and a supply circuit that contains a liquid where the scattering particles are suspended, the scattering particles being configured to scatter a laser radiation emitted by the laser source, said supply circuit being connectable to the first duct of the device.

According to a further aspect, the invention relates to a method for conveying a laser radiation towards a surface that shall be treated by means of the laser radiation, the method comprising the following steps:
arranging a first duct, for supplying a liquid containing scattering particles, adjacent to said surface;
arranging a light guide adjacent to said surface;
supplying liquid through the first duct and wetting the surface with the liquid containing the scattering particles;
emitting a laser beam by means of said light guide in a volume occupied by the supplied liquid;
distributing, through the scattering particles, the laser radiation on the surface wet by the liquid containing the scattering particles.

The method may furthermore comprise the step of sucking the liquid and the scattering particles from said volume.

Further advantageous characteristics and embodiments of the plant and of the method of the invention are described hereafter with reference to the attached drawings, which show a non-limiting practical embodiment, and in the attached claims, forming an integral part of the present description.

### Brief description of the drawings

The invention will be better understood by following the description and the accompanying drawing, which shows non-limiting practical embodiments of the invention. More particularly, in the drawing:
Figure 1 shows a schematic longitudinal cross section of a device according to the invention in a first embodiment;
Figures 2A and 2B show cross sections according to II-II of Figure 1 in two different embodiments;
Figure 3 shows a longitudinal cross section, similar to that of Figure 1, of a further embodiment;
Figures 4 and 5 show longitudinal cross sections of further embodiments of the device; and
Figures 6 and 7 show modes of use of the device.

### Detailed description of embodiments

The following detailed description of the exemplary embodiments refers to the accompanying drawings. The same reference numbers in different drawings identify the same or similar elements. Additionally, the drawings are not necessarily drawn to scale. Also, the following detailed description does not limit the invention. Instead, the scope of the invention is defined by the appended claims.

Reference throughout the specification to "one embodiment" or "an embodiment" or "some embodiments" means that the particular feature, structure or characteristic described in connection with an embodiment is included in at least one embodiment of the subject matter disclosed. Thus, the appearance of the phrase "in one embodiment" or "in an embodiment" or "in some embodiments" in various places throughout the specification is not necessarily referring to the same embodiment(s). Further, the particular features, structures or characteristics may be combined in any suitable manner in one or more embodiments.

Below, specific reference will be made to dental applications, but it should be understood that a device according to the invention can be also used for other medical or veterinary applications, where similar problems of bacterial proliferation occur in areas that are difficult to be accessed by means of usual light guides.

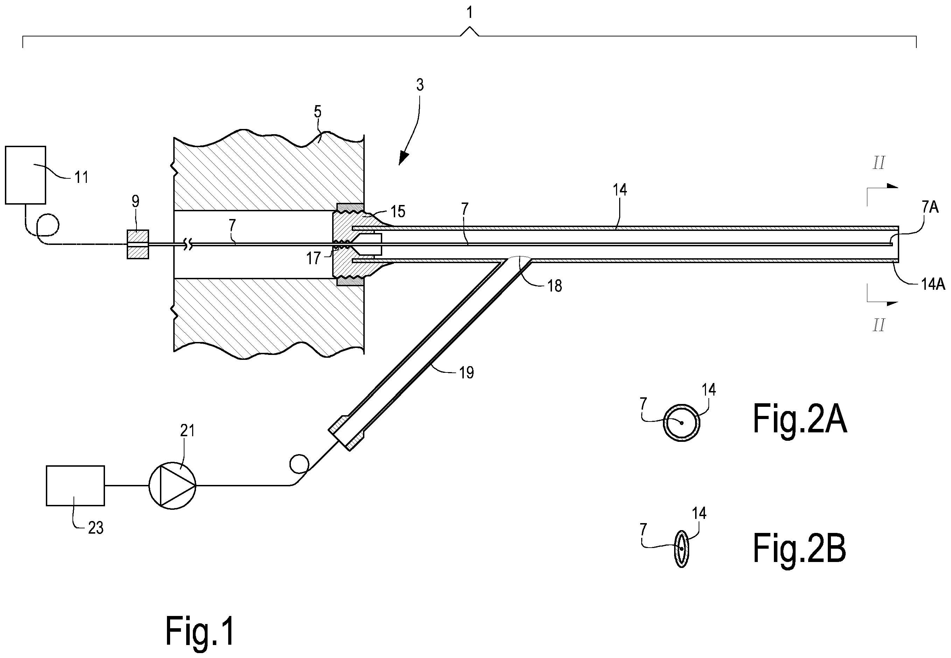

With initial reference to Figure 1, number 1 schematically indicates an equipment according to the invention. Reference number 3 indicates a device for applying the laser radiation to a patient's organ or tissue. In the illustrated embodiment, the device 3 comprises a hand-piece or handle 5, to which a light guide, constituted by one or more optical fibers 7, arrives. Below, reference will be specifically made to an embodiment with only one optical fiber, but it should be understood that this configuration is only a non-limiting example.

The optical fiber 7 may be connected, for example through a connector 9, to a laser source 11 of the equipment 1, schematically shown only in Figure 1. In the embodiment illustrated in Figure 1, the end portion of the optical fiber 7 extends inside a first duct 14, the proximal end whereof is attached to the hand-piece 5 and the distal end 14A whereof is open. The distal end 7A of the optical fiber 7 may be arranged near the open distal end 14A of duct 14. In some embodiments, the position of the distal end 7A of the fiber may be adjusted with respect to the duct 14, so that the distal end may be positioned either exactly in correspondence of the open distal end 14A of the duct 14, or in a back position inside the duct 14, as illustrated in Figure 1, or projecting outside the duct 14, for example by a length varying from some tenths of millimeter to some millimeters.

In some embodiments, the duct 14 may be connected to the hand-piece or handle 5 by means of a screw connection or the like, schematically indicated with 15, in order to allow the duct 14 to be removed and replaced. For hygienic reasons, the duct 14 may be a disposable duct, for example, or it may be removed through the connection 15 in order to be easily sterilized.

As schematically illustrated, for reasons that will be clearly apparent below, a gasket 17 separates the inside of the hand-piece 5 from the inside of the duct 14 by sealingly contacting the surface of optical fiber 7.

The duct 14 is in fluid connection with an inlet port 18, through which a liquid, for example a physiologic solution, may be supplied. The inlet port 18 may be provided where a side duct 19 is connected to the duct 14. In the schematic of Figure 1, number 21 indicates a pump of equipment 1, pumping the liquid from a tank 23 to the side duct 19. The liquid pumped from the tank 23 to the side duct 19 flows along the duct 14 and exits from the open distal end 14A thereof. The gasket 17 prevents the liquid from flowing back towards the inside of the hand-piece 5 along the optical fiber 7.

The liquid flows through the free area between the optical fiber 7 and the inner wall of the duct 14, the cross section whereof may be of various shapes. Figures 2A and 2B show two alternative embodiments of the end part of duct 14. In Figure 2A, the cross section of the duct 14 is substantially round, while in Figure 2B it is substantially elliptical. In both cases, the optical fiber 7 may be arranged approximately at the center of the cross section of the duct and, if necessary, it may be kept in this position by means of suitable radial spacers, not shown. The shape of the cross section of the duct 14 may be variable, and several interchangeable ducts may be provided, for example for different applications. The shape of Figure 2A is particularly useful for root canal treatment and for treating gingival fistulas or spaces between implant pins and bony tissue in case of pin detachment. Vice versa, the shape of Figure 2B is useful for the treatment of gingival pockets or other applications, where the area to be treated can be better accessed by means of a flat device.

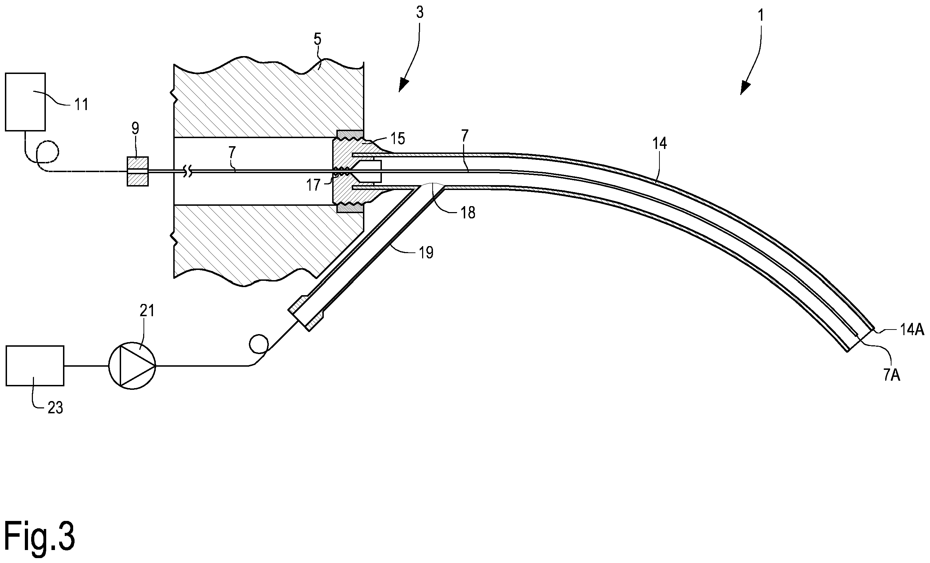

In the embodiment of Figure 1, the duct 14 is rectilinear. In other embodiments, the duct 14 may be curved, as schematically shown in Figure 3, where the same reference numbers indicate equal or equivalent parts to those described with reference to Figure 1.

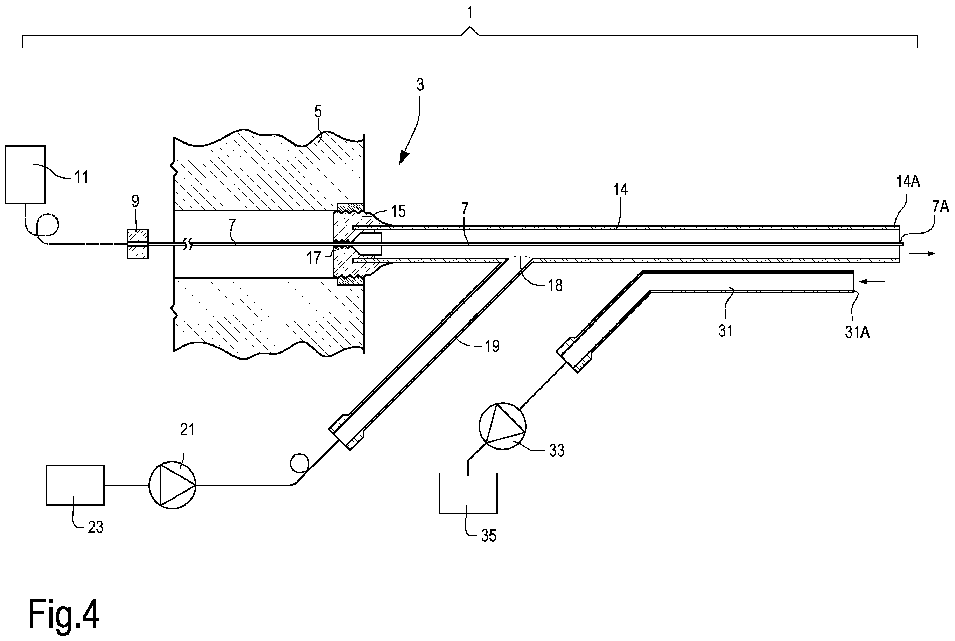

Similarly to Figure 1, Figure 4 shows a schematic of a different embodiment of an equipment according to the invention. The same reference numbers indicate the same or equivalent parts to those described with reference to Figure 1, which will not be described again. In Figure 4, the distal end 7A of the optical fiber 7 is shown in a position slightly projecting from the open distal end 14A of the duct 14. Differently from the embodiment of Figure 1, in Figure 4 the device 3 comprises a suction duct 31 that may be integral with duct 14 and may have an open distal end 31A arranged near the open distal end 14A of the duct 14. In the embodiment of Figure 4, the open distal end 31A of the suction duct 31 is arranged slightly back with respect to the open distal end 14A of the duct 14. The suction duct 31 may be in fluid connection with a suction pump 33 that sucks the liquid through the suction duct 31 and conveys it to a collection tank 33, for purposes that will be better explained below.

With such an arrangement, the liquid supplied by the pump 21 through the duct 14 wets the area to be treated with the device 3 and is then removed from said area thanks to the suction effect of the suction duct 31.

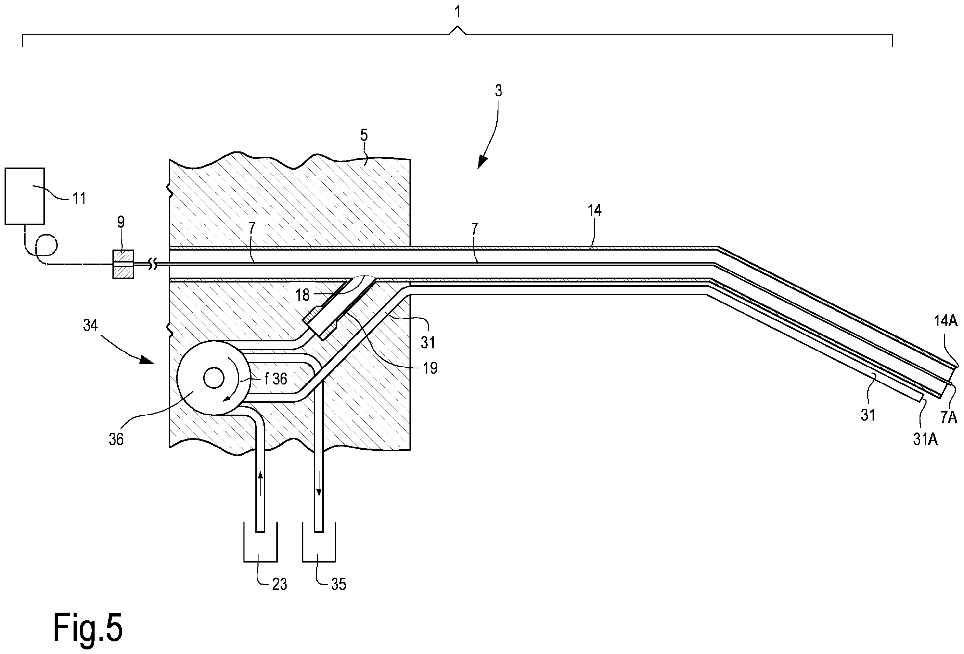

Figure 5 shows a modified embodiment of the device od Figure 4. Equal numbers indicate equal or equivalent parts to those described above with reference to Figures 1 and 4. In the embodiment of Figure 5, the suction duct 31 and the side duct 19 are associated with a single pump 34, for example a peristaltic pump. The pump 34 may be miniaturized and arranged inside the hand-piece 5. In other embodiments, the ducts 19 and 31 may project from the hand-piece 5 and be connected to a pump 34 arranged outside the hand-piece.

In some embodiments, the peristaltic pump 34 may comprise a single rotor acting on two flexible pipes extending around the rotor itself and forming part of the side duct 19 and of the suction duct 31 respectively. The rotor is schematically indicated in Figure 5 with number 36, and the rotation direction thereof is indicated with f36. Thanks to the arrangement of the ducts 19 and 31, the rotation of the single rotor 36 causes the fresh liquid taken from the tank 23 to be pushed into the duct 14 and the exhausted liquid to be sucked through the open distal end 31A of the suction duct 31 and to be discharged into the collection tank 36.

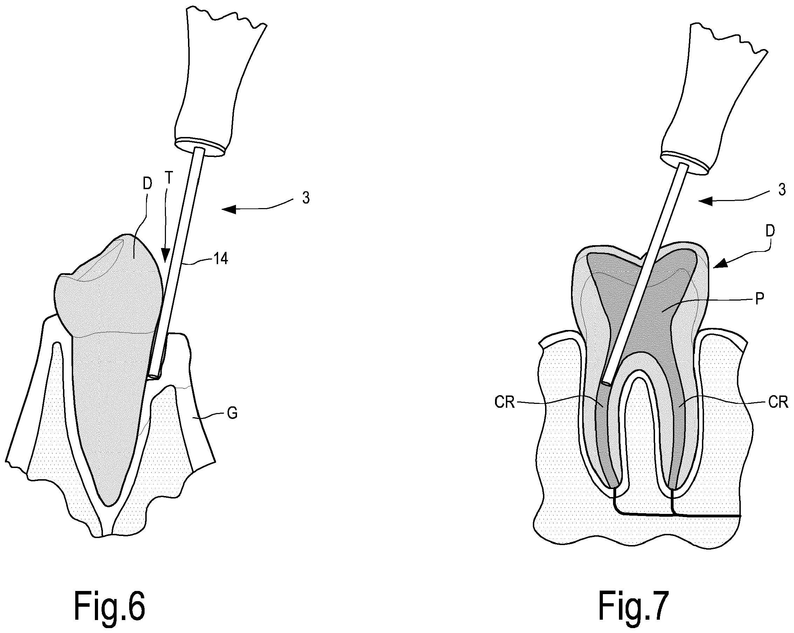

Figure 6 shows a mode of use of the device 3 for treating gingival pockets. D indicates a tooth, and G indicates the gum. T indicates a gingival pocket. The gingival pocket shall be treated to eliminate the microorganisms present on the tissues and to eliminate inflammations. The treatment substantially occurs as described below. The operator puts the distal end 14A of the duct 14 at the entrance of, or inside, the gingival pocket T. In this application, in order to facilitate the positioning of the distal end it is useful to use a device 3 wherein the cross section of the duct 14 is flat at least in the end area, as schematically indicated in Figure 2B.

One or more control buttons may be provided on the hand-piece or handle 5 of device 3 to control the members of the equipment 1, in particular the laser source 11 and the pump(s) 21, 33, 34 according to the device configuration.

Alternatively, the controls, or part of them, may be given through a separate console, one or more pedals or other suitable interface devices or members, not shown.

When the open distal end 14A of the duct 14 has been positioned, the operator starts the treatment cycle. The liquid is dispensed through duct 14 and flows in the area to be treated, in this case the inside of the gingival pocket. The liquid may be any bio-compatible liquid, for example a physiologic solution, or distilled water.

The liquid contains a suspended scattering substance, for example in the form of powder. Powder means, in the present description, any form where the scattering substance is subdivided into particles that are sufficiently small to circulate in the area to be treated, carried by the liquid. The particles may be spherical, approximately spherical, or granular in shape, or they may even have the shape of flakes or any other shape ensuring the desired scattering effect.

In fact, the scattering substance has the function of scattering the laser beam the operator activates during treatment. The laser beam emitted by the source 11 is "fired" from the distal end 7A of the fiber and hits against the scattering particles suspended in the liquid. In fact, the liquid, flowing through the open distal end 14A of the duct 14, fills the volume of the area to be treated, that is, in the case of Figure 6, the gingival pocket T.

In this way, the laser light is scattered by means of the scattering particles suspended in the liquid, and achieves the surrounding tissues, following a complex path, determined by the various reflections on the suspended particles, instead of a rectilinear one. In this way the laser radiation can also achieve non-visible areas of the cavity where the liquid is supplied.

The parameters of the laser radiation are selected to cause sterilization of the surfaces whereon the radiation impinges. In other words, the liquid, thanks to the scattering particles suspended therein, conveys the laser radiation, emitted by means of the fiber 7 and immersed in the liquid, onto all the surfaces wet by the liquid, also onto the surface portions that do not "see" the optical fiber, i.e. that are not on a rectilinear path of the laser beams exiting from the fiber.

The laser radiation may be so selected as to obtain not only tissue sterilization and elimination of the pathogen germs, but also bio-stimulation of the tissues. This is particularly useful and important in odonto-stomathology applications mentioned herein, as it allows a better recovery.

In use, the liquid with the suspended scattering particles starts to be pumped preferably before the laser irradiation, so that the laser beam is scattered from the very beginning and never achieves directly the surrounding tissues. This allows to treat the tissues with the laser not directly, but in a "mediate" manner through the powder substance suspended in the liquid.

When the device is of the type illustrated in Figures 4 or 5, the liquid with the suspended particles is also sucked from the treated area, thus making the treatment more comfortable and less invasive for the patient. In fact, the liquid with the scattering particles is dispensed towards and gradually removed from the treated area, thus avoiding accumulations that are uncomfortable for the patient. Thanks to the suction of the exhausted liquid through the duct 31, i.e. of the liquid already sprayed on the treated area, it is possible to work also with high liquid flows without trouble for the patient. The liquid flow may be metered, for example in order to achieve a constant cooling of the treated area, that otherwise could overheat due to the effect of laser power supplied by the optical fiber 7, with consequent troubles for the patients or even damages to the tissues. The continuous or controlled supply of scattering liquid allows also washing and removal of material that detaches during the treatment. If a suction duct 31 is provided, the debris and residues detached from the structures wet by the liquid are accumulated together with the exhausted liquid and can be eliminated, for example by collecting them in single-use containers or tanks that can be sealed.

Figure 7 illustrates a further use of the device in odonto-stomathology. The device is indicated again with reference number 3. In this example, the device 3 and the equipment 1 are used to treat root canals CR of a tooth D, for example following a devitalization due to a caries that seriously damaged the pulp cavity P. The open distal end 14A of the duct 14 is inserted in the pulp cavity P and in the root canal CR to be treated. The operator starts to supply the liquid containing the scattering particles and then, with a suitable delay, starts to supply the laser beam. The laser radiation achieves the whole surface wet by the liquid containing the scattering particles, even in areas that cannot be achieved by a direct laser beam.

In Figure 7, the duct 14 is curved, as shown for instance in Figure 2. In some embodiments, a kit of interchangeable ducts 14 may be provided, the ducts being different from one another in lengths, outer diameters and curvatures, in order to achieve the inside of root canals of different shape and (cross and/or longitudinal) dimensions.

In some embodiments, the duct 14 may be made of flexible material, so as to adapt to the shape of the root canal.

When a device 3 of the type described herein is used for the treatment of root canals CR, the treatment method can be as follows. The operator opens the tooth D accessing the pulp cavity P and partially or completely removes the soft tissues from the interior of the cavity by means of known mechanical systems. Lastly, the operator performs a final sterilization treatment by means of device 3. The circulation of liquid with the suspended scattering particles allows scattering the light, achieving the whole wet surface, to control the tooth temperature, and to remove any residues of tissue, blood or other debris inside the tooth.

The device 3 and the equipment 1 described above may be also used for other applications, such as the treatment of spaces between the implant pins and the surrounding bone when there are detachments between pin and bony tissue following infections, or the treatment of cavities due to abscesses, infected areas surrounding metal suture points or the like. In any cases, the device 3 with the hydraulic circuit supplying the liquid with the suspended scattering particles and with the laser source and the corresponding radiation conveying system, allows to disinfect or sterilize the tissues also in areas that cannot be accessed directly by means of the laser beam emitted by the optical fiber. Moreover, the laser radiation has a bio-stimulating effect on the tissues, facilitating or accelerating the recovery, for instance by promoting the proliferation of tissue that fills the empty space between bony tissue and pin in the case of implant pin detachment.

In some embodiments, the wavelengths of the laser source 11 usable in the equipment 1 described herein is comprised between about 700 and about 3000 micrometers.

The diameter of the optical fiber 7 or fiber bundle may be comprised between about 50 micrometers and about 1000 micrometers, and preferably between about 200 micrometers and about 400 micrometers.

In advantageous embodiments, the laser average power may be comprised between about 1W and about 30W, preferably between about 2W and about 20W.

The laser radiation may be a continuous radiation or a pulsed radiation. In the second case, the peak power may be comprised, for example, between 100W and 10kW, with impulsive waveforms, the duration whereof is comprised between about 20 nanoseconds and about 1000 microseconds, and repetition frequency which can be comprised between 1 kHz and 20Hz.

The laser source may be, for example, a single source or a multiple source in the wavelength range between about 700 nm and about 3000 nm, suitable for continuous, pulsed or Q-switching emission, for example an Nd:YAG laser.

The scattering powder substance, suspended in the liquid, may be selected from the group consisting of hydroxylapatite, or other bio-compatible powder substances. The scattering substance may have an average grain size comprised, for example, between about 300 nm and about 1500 nm.

Even if, in the embodiments described above, reference has been made to a scattering substance in the form of solid suspended powder or particles, according to other embodiments the scattering particles may be particles of a gaseous scattering substance, dispersed, for instance, in the form of gaseous micro-bubbles, or a liquid substance, in the form of emulsion in water, physiologic solution, or other carrying liquid.

In general, the dimension of the scattering particles suspended in the liquid and the quantity of particles per liquid volume unit may be proportioned to support a laser diffusion allowing to achieve, at a maximum work distance from the fiber distal end 7A, an intensity not lower than 40%, preferably not lower than 50%, more preferably not lower than 70% of the initial power, near the emitting surface of the fiber. For example, the maximum work distance may be comprised between about 7 mm and about 28 mm, according to the application.

The laser source may be a Nd:YAG laser generated by a solid-state source operating in both continuous and pulsed mode in the free-running mode or, if necessary, in the Q-switching mode. The high peak powers achievable in Q-switching mode and, partially, in pulsed mode, generate a supplementary photomechanical effect contributing to the removal of the bacterial film in the treated area.

It is understood that the drawing only shows an example provided by way of a practical arrangement of the invention, which can vary in forms and arrangement without however departing from the scope of the concept underlying the invention. Any reference numerals in the appended claims are provided to facilitate reading of the claims with reference to the description and to the drawing, and do not limit the scope of protection represented by the claims.

## Claims

1. A medical equipment (1)
for tissue laser treatments, comprising:
- a device (3) comprised of: a first duct (14) having a proximal end, associated with a hand-piece, and an open distal end (14A), the first duct (14) communicating with an inlet port for a liquid containing scattering particles and configured to dispense liquid containing scattering particles in an area to be treated; a light guide (7) arranged and configured to convey a laser radiation near or at the open distal end (14A) of the first duct (14);
- a laser source (11) connectable to the light guide (7);
- a supply circuit that contains a liquid where the scattering particles are suspended, the scattering particles being configured to scatter a laser radiation emitted by the laser source (11) and emitted by the light guide (7), said supply circuit being connectable to the first duct (14) of the device (3).

2. Equipment (1) according to claim 1, comprising a source (23) of a liquid, in which the scattering particles are suspended, the source (23) in fluid communication with the first duct (14).

3. Equipment (1) according to claim 1 or 2, wherein the light guide (7) is arranged inside the first duct (14) and extends longitudinally there along, up to a position adjacent to the open distal end (14A) of the first duct (14).

4. Equipment (1) according to claim 1 or 2 or 3, wherein the light guide (7) comprises at least one optical fiber (7).

5. Equipment (1) according to one or more of the preceding claims, wherein the light guide (7) comprises a connector (9) for connection to a laser radiation source (11).

6. Equipment (1) according to one or more of the preceding claims, wherein the cross section of the distal end of the first duct (14) is approximately circular or approximately elliptical.

7. Equipment (1) according to one or more of the preceding claims, wherein the position of the light guide (7) with respect to the first duct (14) is adjustable according to a longitudinal direction of the first duct (14).

8. Equipment (1) according to one or more of the preceding claims, comprising a second suction duct (31) to remove liquid from a treated area, the second suction duct (31) having an inlet arranged near the open distal end (14A) of the first duct (14).

9. Equipment (1) according to one or more of the preceding claims, wherein at least one pump (21) is arranged in the hand-piece to pump said liquid containing scattering particles.

10. Equipment (1) according to claim 9 when depending at least on claim 6, wherein said at least one pump (34) is configured and arranged to pump liquid containing scattering particles into the first duct (14) and to suck liquid containing scattering particles through the second suction duct (31).

11. Equipment (1) according to any one of the preceding claims, wherein the source (11) emits laser radiation in a wavelength range comprised between about 700 nm and about 3000 nm.

12. Equipment (1) according to any one of the preceding claims, wherein the parameters of the laser radiation scattered by the scattering particles are selected to have a sterilizing effect or a bio-stimulating effect.

## Patentansprüche

1. Medizinische Ausrüstung (1) für Gewebe-Laserbehandlungen mit:
einer Vorrichtung (3) mit: einer ersten Leitung (14) mit einem proximalen Ende, das einem Handgriff zugeordnet ist, und einem offenen distalen Ende (14A), wobei die erste Leitung (14) mit einem Einlass für Flüssigkeit kommuniziert, die Streupartikel enthält, und ausgebildet ist, um Flüssigkeit, die Streupartikel enthält, in einen zu behandelnden Bereich abzugeben, einem Lichtleiter (7), der angeordnet und ausgebildet ist, um Laserstrahlung in die Nähe oder zu dem offenen distalen Ende (14A) der ersten Leitung (14) zu leiten,
einer Laserquelle (11), die mit dem Lichtleiter (7) verbindbar ist,
einem Zufuhrkreis, der eine Flüssigkeit enthält, in der die Streupartikel suspendiert sind, wobei die Streupartikel ausgebildet sind, um Laserstrahlung, die durch die Laserquelle (11) abgegeben wird und durch den Lichtleiter (7) emittiert wird, zu streuen, wobei der Zufuhrkreis mit der ersten Leitung (14) der Vorrichtung (3) verbindbar ist.

2. Ausrüstung (1) nach Anspruch 1 mit einer Quelle (23) einer Flüssigkeit, in der die Streupartikel suspendiert sind, wobei die Quelle (23) in Fluidverbindung mit der ersten Leitung (14) steht.

3. Ausrüstung (1) nach Anspruch 1 oder 2, wobei der Lichtleiter (7) innerhalb der ersten Leitung (14) angeordnet ist und sich ihr entlang längs zu einer Position angrenzend an das offene distale Ende (14A) der ersten Leitung (14) erstreckt.

4. Ausrüstung (1) nach Anspruch 1 oder 2 oder 3, wobei der Lichtleiter (7) mindestens eine optische Faser (7) aufweist.

5. Ausrüstung (1) nach einem oder mehreren der vorstehenden Ansprüche, wobei der Lichtleiter (7) einen Verbinder (9) zur Verbindung mit einer Laser-Strahlungsquelle (11) aufweist.

6. Ausrüstung (1) nach einem oder mehreren der vorstehenden Ansprüche, wobei der Querschnitt des distalen Endes der ersten Leitung (14) etwa kreisförmig oder etwa elliptisch ist.

7. Ausrüstung (1) nach einem oder mehreren der vorstehenden Ansprüche, wobei die Position des Lichtleiters (7) mit Bezug auf die erste Leitung (14) entsprechend einer Längsrichtung der ersten Leitung (14) einstellbar ist.

8. Ausrüstung (1) nach einem oder mehreren der vorstehenden Ansprüche mit einer zweiten Saugleitung (31) zum Entfernen von Flüssigkeit aus einem behandelten Bereich, wobei die zweite Saugleitung (31) einen Einlass aufweist, der in der Nähe des offenen distalen Endes (14A) der ersten Leitung (14) angeordnet ist.

9. Ausrüstung (1) nach einem oder mehreren der vorstehenden Ansprüche, wobei mindestens eine Pumpe (21) in dem Handgriff angeordnet ist, um die Flüssigkeit, die Streupartikel enthält, zu pumpen.

10. Ausrüstung (1) nach Anspruch 9, wenn abhängig von mindestens Anspruch 6, wobei die mindestens eine Pumpe (34) ausgebildet und angeordnet ist, um Flüssigkeit, die Streupartikel enthält, in die erste Leitung (14) zu pumpen und Flüssigkeit, die Streupartikel enthält, durch die zweite Saugleitung (31) anzusaugen.

11. Ausrüstung (1) nach einem der vorstehenden Ansprüche, wobei die Quelle (11) Laserstrahlung in einem Wellenlängenbereich zwischen etwa 700 nm und etwa 3000 nm emittiert.

12. Ausrüstung (1) nach einem der vorstehenden Ansprüche, wobei die Parameter der Laserstrahlung, die durch die Streupartikel gestreut wird, ausgewählt sind, um einen sterilisieren Effekt oder einen bio-stimulierenden Effekt zu haben.

## Revendications

1. Un équipement médical (1) pour les traitements au laser de tissus, comprenant :
- un dispositif (3) constitué de : un premier conduit (14) ayant une extrémité proximale, associé avec une pièce à main, et une extrémité distale ouverte (14A), le premier conduit (14) communiquant avec un orifice d'entrée pour un liquide contenant des particules diffusantes et étant configuré pour délivrer un liquide contenant des particules diffusantes dans une zone à traiter ; un guide de lumière (7) agencé et configuré pour diriger une radiation laser près de ou au niveau de l'extrémité distale ouverte (14A) du premier conduit (14) ;
- une source laser (11) pouvant être connectée au guide de lumière (7) ;
- un circuit d'approvisionnement qui contient un liquide dans lequel les particules diffusantes sont en suspension, les particules diffusantes étant configurées pour diffuser une radiation laser émise par la source laser (11) et émise par le guide de lumière (7), ledit circuit d'approvisionnement pouvant être connecté au premier conduit (14) du dispositif (3).

2. Equipement (1) selon la revendication 1, comprenant une source (23) d'un liquide dans lequel les particules diffusantes sont en suspension, la source (23) étant en communication fluidique avec le premier conduit (14).

3. Equipement (1) selon la revendication 1 ou 2, dans lequel le guide de lumière (7) est agencé à l'intérieur du premier conduit (14) et s'étend longitudinalement le long de celui-ci, jusqu'à une position adjacente à l'extrémité distale ouverte (14A) du premier conduit (14).

4. Equipement (1) selon la revendication 1 ou 2 ou 3, dans lequel le guide de lumière (7) comprend au moins une fibre optique (7).

5. Equipement (1) selon l'une ou plusieurs des revendications précédentes, dans lequel le guide de lumière (7) comprend un connecteur (9) pour une connexion à une source de radiation laser (11).

6. Equipement (1) selon l'une ou plusieurs des revendications précédentes, dans lequel la section transversale de l'extrémité distales du premier conduit (14) est approximativement circulaire ou approximativement elliptique.

7. Equipement (1) selon l'une ou plusieurs des revendications précédentes, dans lequel la position du guide de lumière (7) par rapport au premier conduit (14) est réglable suivant une direction longitudinale du premier conduit (14).

8. Equipement selon l'une ou plusieurs des revendications précédentes, comprenant un second conduit d'aspiration (31) pour retirer du liquide d'une zone traitée, ce second conduit d'aspiration (31) ayant une entrée agencée près de l'extrémité distale ouverte (14A) du premier conduit (14).

9. Equipement (1) selon l'une ou plusieurs des revendications précédentes, dans lequel au moins une pompe (21) est agencée dans la pièce à main pour pomper ledit liquide contenant des particules diffusantes.

10. Equipement (1) selon la revendication (9) lorsque celle-ci dépend au moins de la revendication 6, dans lequel ladite ou lesdites pompe(s) (34) est(sont) configurée(s) et agencée(s) pour pomper du liquide contenant des particules diffusantes dans le premier conduit (14) et pour aspirer du liquide contenant des particules diffusantes à travers le second conduit d'aspiration (31).

11. Equipement (1) selon l'une quelconque des revendications précédentes, dans lequel la source (11) émet une radiation laser ayant une plage de longueurs d'onde comprise entre environ 700 nm et environ 3000 nm.

12. Equipement (1) selon l'une quelconque des revendications précédentes, dans lequel les paramètres de la radiation laser diffusée par les particules diffusantes sont sélectionnés pour avoir un effet stérilisant ou un effet biostimulant.
